# EUROPEAN PATENT APPLICATION

(11) **EP 3 138 545 A1**
(43) Date of publication of application: **08.03.2017**
(21) Application number: 15786632.8
(22) Date of filing: 20.01.2015
(51) Int. Cl.: A61F 13/496

(54) **UNDERPANTS-TYPE ABSORBENT ARTICLE**

(30) Priority: 28.04.2014 JP 2014092701
(71) Applicant: Unicharm Corporation, Ehime 799-0111 (JP)
(72) Inventor: SHIMAZU, Takeshi, Kanonji-shi Kagawa 769-1602 (JP); YAMAMOTO, Hiroki, Kanonji-shi Kagawa 769-1602 (JP); NASHIKI, Kento, Kanonji-shi Kagawa 769-1602 (JP)
(74) Representative: Dolleymores
(86) International application number: PCT/JP2015/051383
(87) International publication number: WO 2015/166671

(57) **Abstract**

An underpants-type absorbent article configured with an absorbent article having an abdomen side part that is to be arranged on an abdomen side of a wearer, a back side part that is to be arranged on a back side of the wearer and a crotch part that is to be arranged to a crotch of the wearer when worn, the absorbent article being welded at respective horizontally outward end parts of the abdomen side part and the back side part when the absorbent article is in a state folded into two at the crotch part with the abdomen side part overlapping the back side part to form a body encircling opening and a pair of leg encircling openings at mutually different locations along a vertical direction that intersects the horizontal direction, the underpants-type absorbent article including
a plurality of welded parts that are formed at the end part in a non-continuous welding pattern along at least the vertical direction; and
an inner area and an outer area that are included in the end part, the inner area being located horizontally inward and the outer area being located horizontally outward with respect to the inner area; wherein
the welded parts are formed to a predetermined range along the vertical direction, the predetermined range starting from the body encircling opening of the end part, such that a vertical dimension of a space between vertically adjacent welded parts in the inner area is made larger than the vertical dimension of a space between the vertically adjacent welded parts in the outer area, and
the welded parts each has on the body encircling opening side a slanted shape portion, the slanting portion slanting toward the leg encircling opening in the vertical direction as approaching to an outside along the horizontal direction, the welded parts being formed in pluralities and arranged along the vertical direction at the inner area in the predetermined range.

## Description

### [Technical Field]

The present invention relates to underpants-type absorbent articles that absorb excreted liquid such as urine.

### [Background Art]

Underpants-type disposable diapers have been conventionally used as absorbent articles that absorb excreted liquid such as urine. This type of diaper includes an abdomen side part which comes to the abdomen side of the wearer, a back side part which comes to the back side of the wearer and a crotch part which comes to the crotch part of the wearer when the diaper is worn. And this diaper has the horizontal end parts of the abdomen side part and the back side part welded with the diaper being folded into two at the crotch part with the abdomen side part and the back side part laid over the other. And hereby, an underpants-type diaper is formed creating a body encircling opening and a pair of leg encircling openings to be provided to the user.

### [Citation List]

### [Patent Literature]

[PTL 1] Japanese Patent Application Laid-open Publication No. 2008-253633

### [Summary of Invention]

### [Technical Problem]

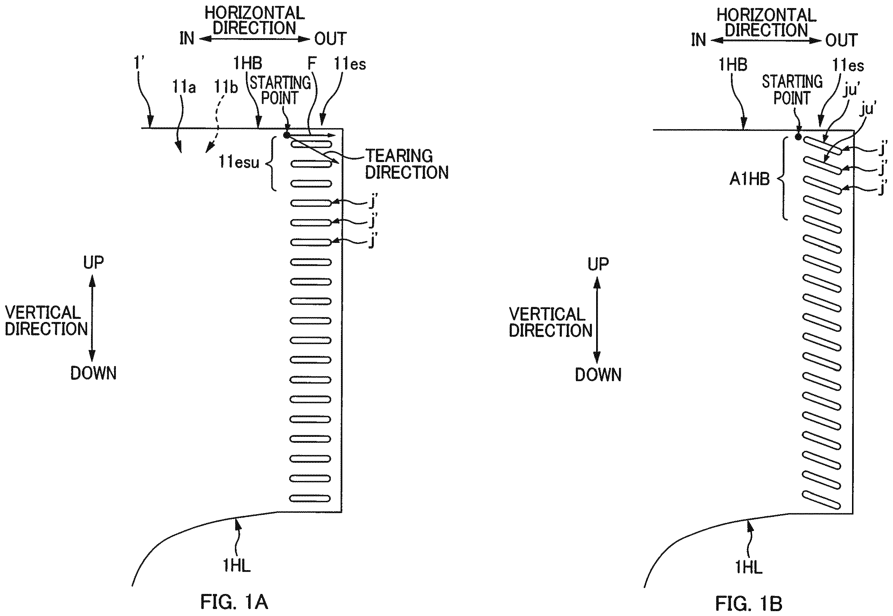

Fig. 1A illustrates a schematic enlarged view of the horizontal end part 11es of the underpants-type diaper 1'. As illustrated in Fig. 1A, this diaper 1' has formed a plurality of welded parts j', j' ... to each of the horizontal end parts 11es of the aforementioned abdomen side part 11a and the back side part 11b by welding the aforementioned abdomen side part 11a and the back side part 11b. And in this example, the shape of the welded parts j' are in rectangular shapes long in the horizontal direction and the welded parts j' are provided aligned in a row from the body encircling opening 1HB side to the leg encircling opening 1HL side.

And when this underpants-type diaper 1' is taken off from the wearer after excretion, it is common that the abdomen side part 11a and the back side part 11b are separated by tearing the aforementioned end part 11es from the inner side toward the outer side in the horizontal direction starting from the body encircling opening 1HB.

Here, when the tearing direction is viewed in a microscopic manner, the tearing direction is directed outward in the horizontal direction while being directed downward being the direction toward the leg encircling opening 1HL along the vertical direction as illustrated in Fig. 1A. For such reason, when the force F that is directed outward along the horizontal direction and imparted to the body encircling opening 1HB by a person' s hand, the force is naturally guided toward the above described tearing direction at this end part 11es, and this is recognized to allow an easy tearing of the end part 11es. Additionally, since a large force F is required at particularly the start of the tearing, the start of the tearing is important. And when the tearing works at the start, the tearing can be accomplished with the created momentum until the end without a problem in most of the cases.

Meanwhile, it is common that the strength of the welded parts j' are stronger than their surrounding parts and therefore, the force required for tearing concentrates on mainly the borders between the welded parts j' and their surrounding parts for the force to act easily. In other words, the force is likely to concentrate and act on the circling edge parts of the welded parts j'. And therefore, even if the shape of the body encircling opening 1HB side parts ju' of the welded parts j' were in a slanted shape slanting downward in the vertical direction toward the leg encircling opening 1HL side as approaching horizontally outward as illustrated in Fig. 1B, the outward force F along the aforementioned horizontal direction imparted by the person' s hand with the body encircling opening 1HB as the starting point can be directed quickly and naturally toward the direction of the aforementioned tearing direction so that an excellent tearing performance is recognized to be achieved. Additionally, as mentioned above, a large force F is required particularly at the starting of the tearing so that to form the welded parts j', j' ... at the area A1HB proximate the body encircling opening 1HB' of the aforementioned end part 11es in the aforementioned slanted shapes is recognized to be effective.

However, such configuration allowing an excellent tearing performance may have the aforementioned end part 11es be torn carelessly by an external force that may inevitably act when wearing the diaper.

The present invention has been made in view of the aforementioned conventional issues and a purpose thereof is to keep the end part, having the abdomen side part and the back side part welded, from being inevitably torn when wearing the diaper while the end part is enabled to be easily torn with the body encircling opening as the starting point.

### [Solution to Problem]

In order to address the above problem, a primary aspect of the invention is directed to an underpants-type absorbent article configured with an absorbent article having an abdomen side part that is to be arranged on an abdomen side of a wearer, a back side part that is to be arranged on a back side of the wearer and a crotch part that is to be arranged to a crotch of the wearer when worn, the absorbent article being welded at respective horizontally outward end parts of the abdomen side part and the back side part when the absorbent article is in a state folded into two at the crotch part with the abdomen side part overlapping the back side part to form a body encircling opening and a pair of leg encircling openings at mutually different locations along a vertical direction that intersects the horizontal direction, the underpants-type absorbent article including:
a plurality of welded parts that are formed at the end part in a non-continuous welding pattern along at least the vertical direction; and
an inner area and an outer area that are included in the end part, the inner area being located horizontally inward and the outer area being located horizontally outward with respect to the inner area; wherein
the welded parts are formed to a predetermined range along the vertical direction, the predetermined range starting from the body encircling opening of the end part, such that a vertical dimension of a space between vertically adjacent welded parts in the inner area is made larger than the vertical dimension of a space between the vertically adjacent welded parts in the outer area, and
the welded parts each has on the body encircling opening side a slanted shape portion, the slanting portion slanting toward the leg encircling opening in the vertical direction as approaching to an outside along the horizontal direction, the welded parts being formed in pluralities and arranged along the vertical direction at the inner area in the predetermined range.

Other features of the present invention will become clear from the description of the present specification with reference to the accompanying drawings.

### [Advantageous Effects of Invention]

According to the present invention, the end part where the abdomen side part and the back side part are welded can be kept from being inevitably torn when wearing the diaper while the end part is allowed to be easily torn with the body encircling opening as the starting point.

### [Brief Description of the Drawings]

[Fig. 1A]
   Fig. 1A is a schematic enlarged view of the horizontal end part 11es of a conventional diaper 1'.
[Fig. 1B]
   Fig. 1B is a schematic enlarged view of the end part 11es exhibiting excellent tearing performance.
[Fig. 2]

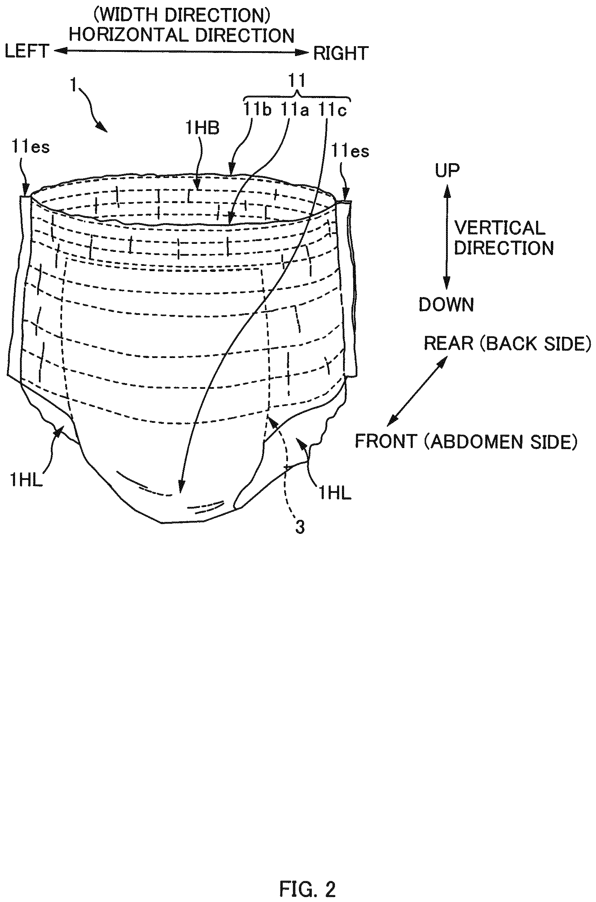

   Fig. 2 is a schematic perspective view of the underpants-type disposable diaper 1 shown as an example of the underpants-type absorbent article according to the present embodiment.
[Fig. 3A]

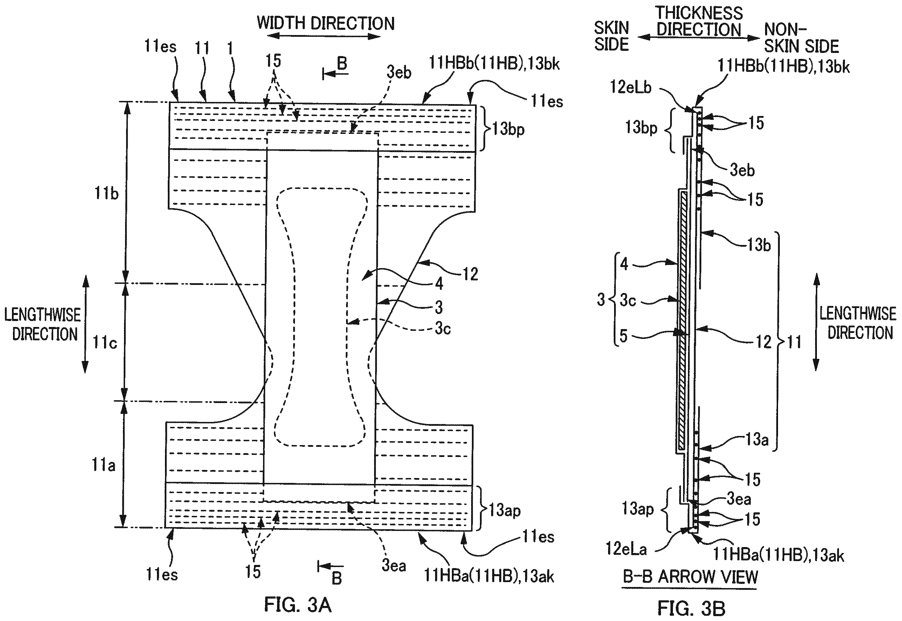

   Fig. 3A is a schematic planar view of the opened state diaper 1 seen from the skin side.
[Fig. 3B]
   Fig. 3B is a schematic view taken along line B-B in Fig. 3A.
[Fig. 4]

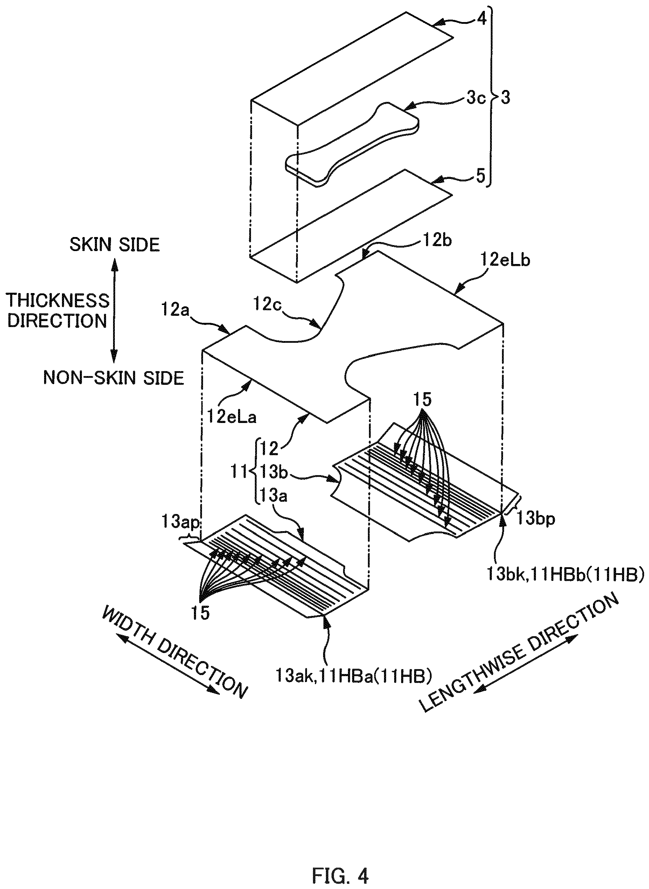

   Fig. 4 is a schematic exploded perspective view of the diaper 1 in an opened state.
[Fig. 5]

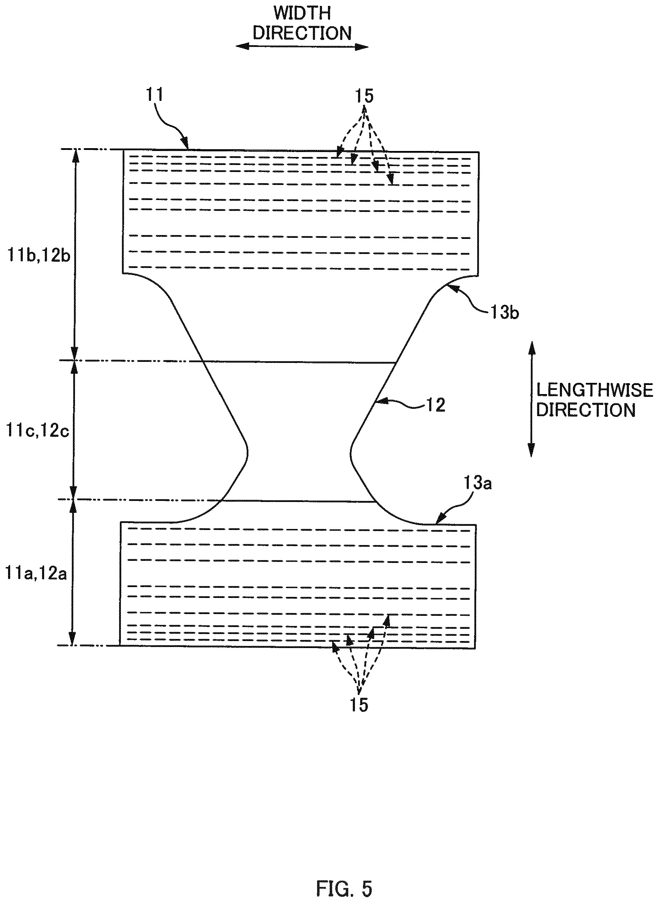

   Fig. 5 is a schematic planar view of an opened state outer covering sheet 11 seen from the non-skin side.
[Fig. 6]

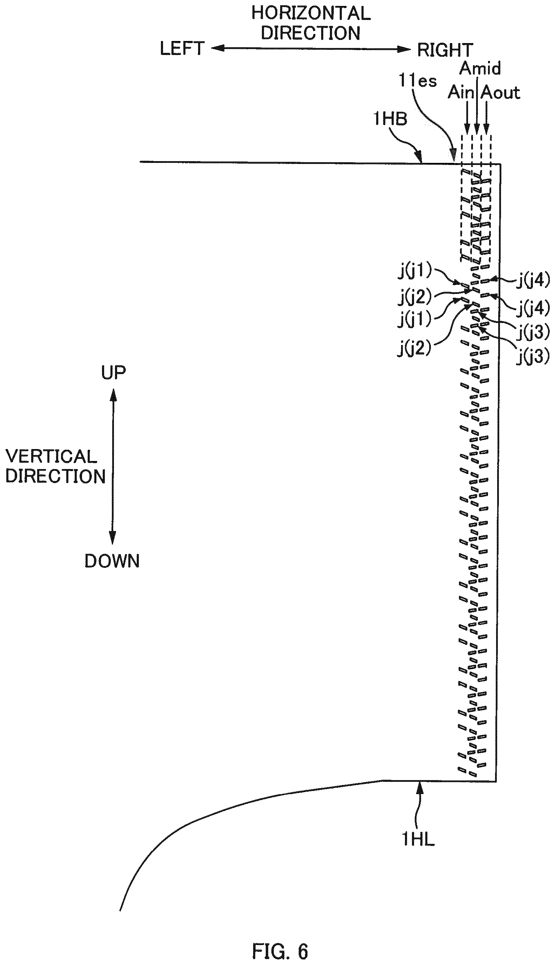

   Fig. 6 is an explanatory view of the welding pattern showing the side end part 11es enlarged.
[Fig. 7A]

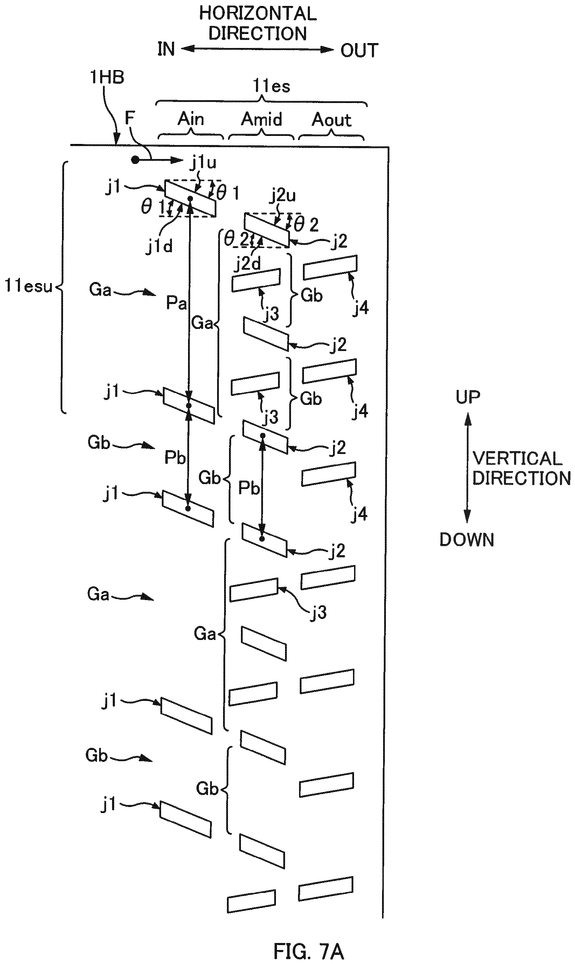

   Fig. 7A is an enlarged view of an upper part of a welding pattern.
[Fig. 7B]

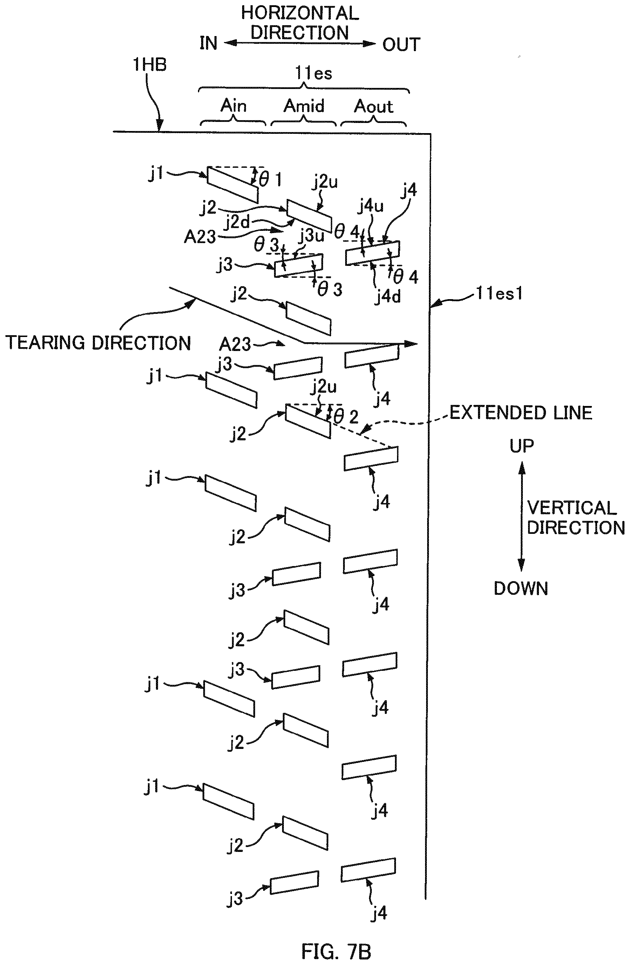

   Fig. 7B is an enlarged view of an upper part of a welding pattern.
[Fig. 8A]

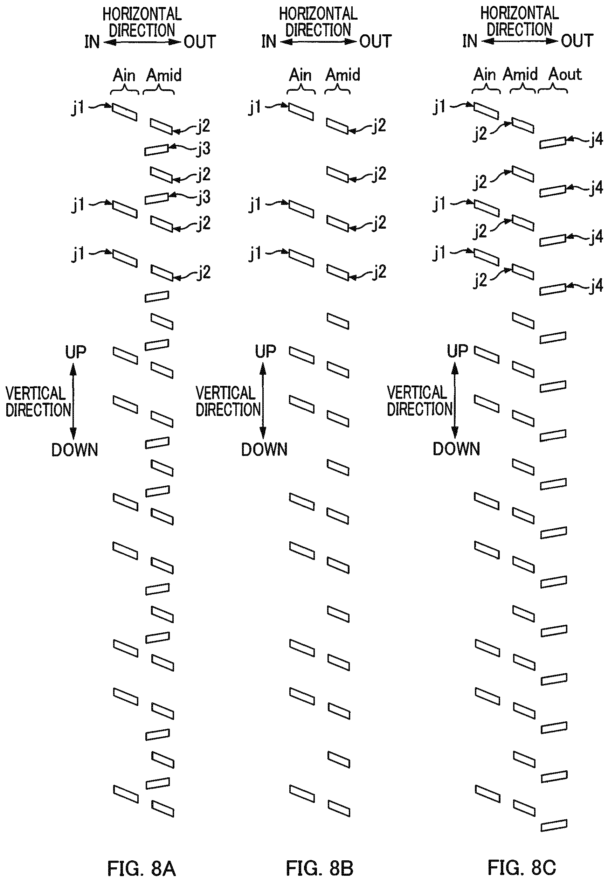

   Fig. 8A is an explanatory view of another welding pattern.
[Fig. 8B]
   Fig. 8B is an explanatory view of another welding pattern.
[Fig. 8C]
   Fig. 8C is an explanatory view of another welding pattern.
[Fig. 9A]

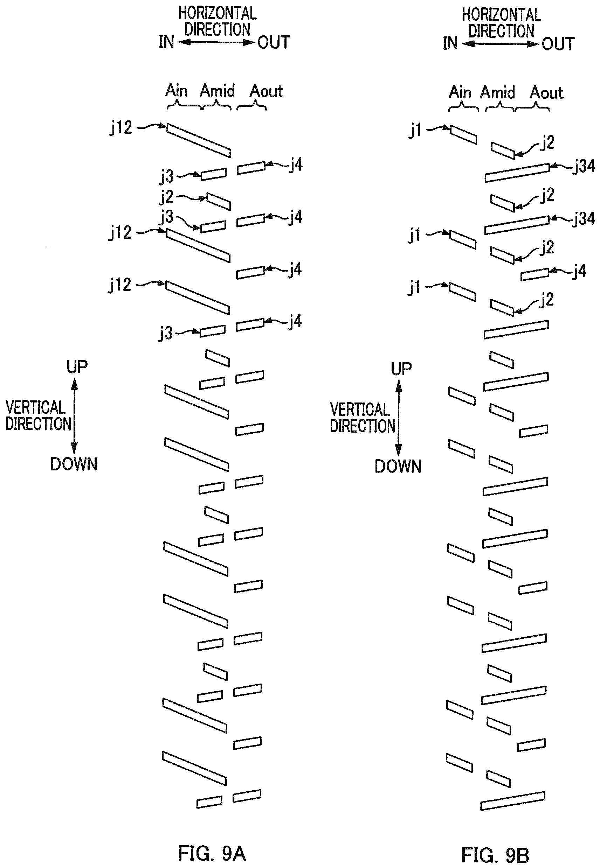

   Fig. 9A is an explanatory view of another welding pattern.
[Fig. 9B]
   Fig. 9B is an explanatory view of another welding pattern.
[Fig. 10]

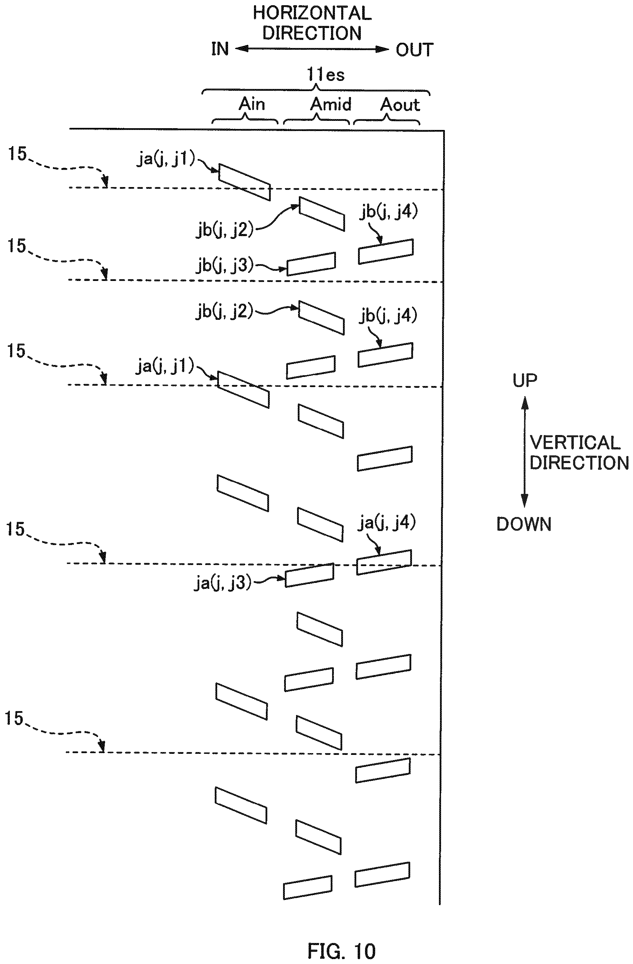

   Fig. 10 is an enlarged view of an upper part of a welding pattern.
[Fig. 11]

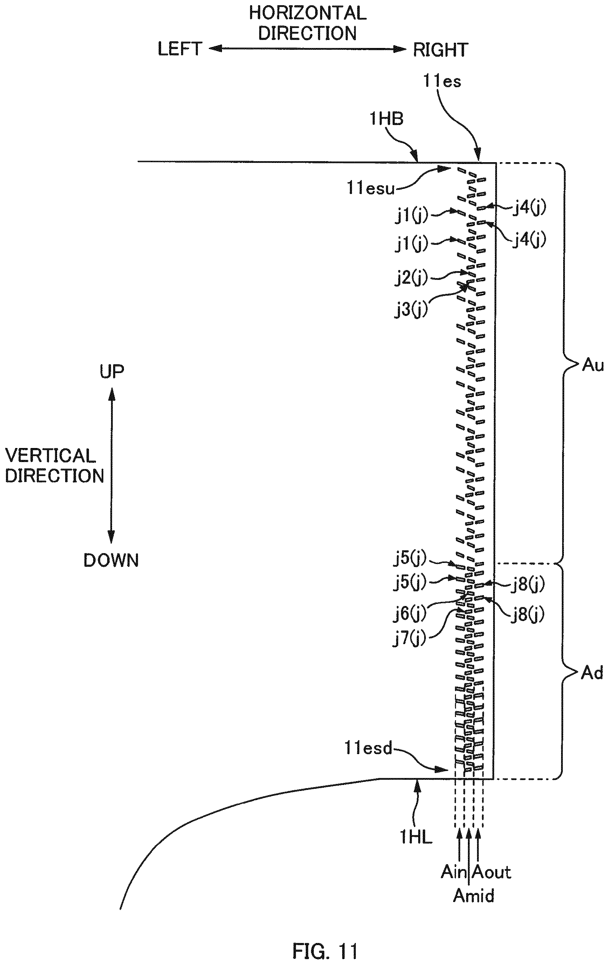

   Fig. 11 is an explanatory view of a modified example of the welding pattern.
[Fig. 12]

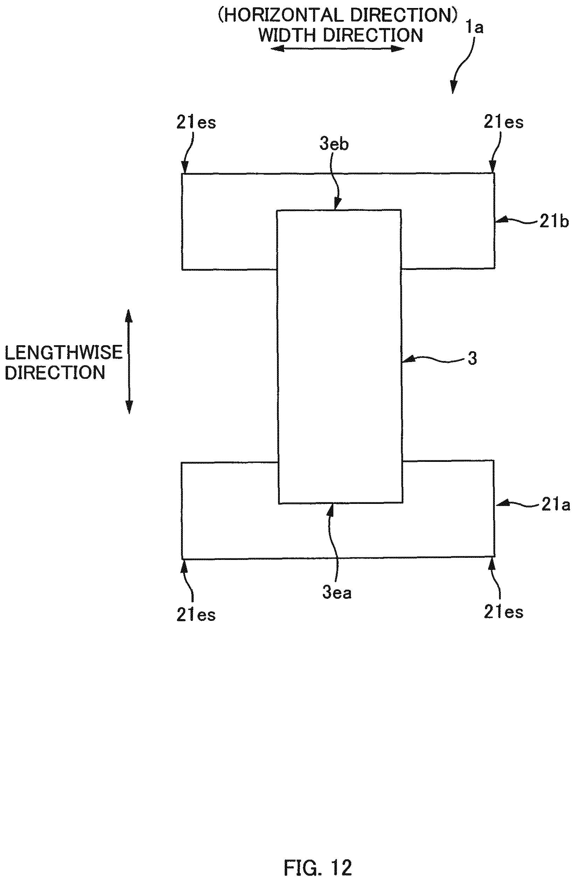

   Fig. 12 is a schematic planar view of an opened state three-pieced type diaper seen from the skin side.

### [Description of Embodiments]

At least the following matters will become clear from the description of the present specification with reference to the accompanying diagrammatic drawings.

Disclosed is an absorbent article featured to include an underpants-type absorbent article configured with an absorbent article having an abdomen side part that is to be arranged on an abdomen side of a wearer, a back side part that is to be arranged on a back side of the wearer and a crotch part that is to be arranged to a crotch of the wearer when worn, the absorbent article being welded at respective horizontally outward end parts of the abdomen side part and the back side part when the absorbent article is in a state folded into two at the crotch part with the abdomen side part overlapping the back side part to form a body encircling opening and a pair of leg encircling openings at mutually different locations along a vertical direction that intersects the horizontal direction, the underpants-type absorbent article including:
a plurality of welded parts that are formed at the end part in a non-continuous welding pattern along at least the vertical direction; and
an inner area and an outer area that are included in the end part, the inner area being located horizontally inward and the outer area being located horizontally outward with respect to the inner area; wherein
the welded parts are formed to a predetermined range along the vertical direction, the predetermined range starting from the body encircling opening of the end part, such that a vertical dimension of a space between vertically adjacent welded parts in the inner area is made larger than the vertical dimension of a space between the vertically adjacent welded parts in the outer area, and
the welded parts each has on the body encircling opening side a slanted shape portion, the slanting portion slanting toward the leg encircling opening in the vertical direction as approaching to an outside along the horizontal direction, the welded parts being formed in pluralities and arranged along the vertical direction at the inner area in the predetermined range.

According to such an underpants-type absorbent article, the absorbent article can have the above described end part easily torn from the body encircling opening when taking the absorbent article off from a wearer, while being kept from having the above described end part inevitably torn when wearing the absorbent article. Details are as follows.

Firstly, the welded part located at the above described inner area at the range adjacent to the body encircling opening being the above described predetermined range, includes at the body encircling opening side a slanted shape portion where the slanting portion slants toward the leg encircling opening in the vertical direction as approaching to the outside along the horizontal direction. Therefore, an outward facing force acting along the horizontal direction imparted to the body encircling opening can be quickly guided toward the previously discussed tearing direction based on this slanted shape to thereby allow this end part to be easily torn in the horizontal direction from the inside toward the outside.

Meanwhile, with respect to the welded part formed at the range adjacent to the body encircling opening being the above described predetermined range, the vertical dimension of the space between the vertically adjacent welded parts in the inner area is made larger than the vertical dimension of the space between the vertically adjacent welded parts in the outer area. Hereby, the above described space in the inner area being large can assure an excellent tearing performance at the start of tearing at the body encircling opening while the above described space in the outer area being small can effectively complement the strength of the above described end part. And hereby, this end part can be kept from being inevitably torn when wearing the absorbent article.

It is preferable that in an underpants-type absorbent article,
the outer area includes a middle area adjacent to the inner area,
and when the welded part including the slanted shape portion is regarded as a first welded part,
a second welded part and a third welded part are provided to the middle area,
the second welded part includes on the leg encircling opening side the slanted shape portion, the slanting portion slanting toward the leg encircling opening in the vertical direction as approaching to an outside along the horizontal direction,
the third welded part includes on the body encircling opening side the slanted shape portion, the slanting portion slanting toward the body encircling opening in the vertical direction as approaching to an outside along the horizontal direction, and
the second welded part and the third welded part associate with each other to partition in the middle area, a taper shaped area that is narrowed in the vertical direction as approaching to the outside along the horizontal direction.

According to such an underpants-type absorbent article, the above described second welded part and the third welded part provided in the middle area associate with each other to partition in this middle area, a taper shaped area that is narrowed in the vertical direction as approaching to the outside along the horizontal direction. This taper shaped area being a non-welded part that is not welded has a lower strength compared to the welded part so that the tearing direction is guided to advance through this taper shaped area. And as a result, this tearing direction is changed to take a route that is substantially parallel to the horizontal direction. Hereby, the above described end part can be torn taking the shortest route which comes closest to the horizontally outward edge part at this end part and thus this end part can be torn easily.

Further, the second welded part and the third welded part that are formed in the middle area effectively contribute to make the space between the vertically adjacent welded parts narrower than the interval between the vertically adjacent welded parts in the inner area.

It is preferable that in an underpants-type absorbent article,
the second welded part includes on the body encircling opening side the slanted shape portion, the slanting portion slanting toward the leg encircling opening in the vertical direction as approaching to the outside along the horizontal direction and
the slanted shape portion is provided along the slanted shape portion included on the body encircling opening side of the first welded part.

According to such an underpants-type absorbent article, the second welded part in the middle area has the above described slanted shape portion on the body encircling opening side and this slanted shape portion is provided along the slanted shape portion included on the body encircling opening side of the first welded part in the inner area. Hereby, the guiding of the above described force toward the tearing direction based on the first welded part in the inner area can be quickly taken over by the second welded part in the inner area. Therefore, the above described guiding can be quickly taken over in also this middle area. And as a result, this end part can be further easily torn.

It is preferable that in an underpants-type absorbent article, the slanted shape portion included on the body encircling opening side of the second welded part has a shape same as that of the slanted shape portion included on the body encircling opening side of the first welded part, and the second welded part and the first welded part are provided to be arranged along the horizontal direction while being inclined away from the horizontal direction such that the mutual slanted shape portions are aligned in a straight line.

According to such an underpants-type absorbent article, the guiding of the above described force toward the tearing direction that is based on the first welded part in the inner area can be further quickly taken over by the second welded part in the middle area. Therefore, the above described guiding can be quickly taken over in also this middle area. And as a result, this end part can be furthermore easily torn.

It is preferable that in an underpants-type absorbent article, the outer area includes an end area located horizontally outward with respect to the middle area,
the end area is provided with a fourth welded part,
the fourth welded part includes on the body encircling opening side the slanted shape portion, the slanting portion slanting toward the body encircling opening in the vertical direction as approaching to an outside along the horizontal direction, and
the fourth welded part is provided to intersect with a line extending from the slanted shape portion included on the body encircling opening side of the second welded part.

According to such an underpants-type absorbent article, the fourth welded part in the end area is provided to intersect with the line extending from the slanted shape portion of the second welded part in the middle area. Therefore, when tearing the above described end part, the tearing momentum along the tearing direction created at the inner area and the middle area can be blocked at the end area to relieve this momentum. As a result, a person can tear the end part in the horizontal direction with an appropriate momentum as a whole.

It is preferable that in an underpants-type absorbent article, an inclination of the slanted shape portion of the fourth welded part is more gentle with respect to an inclination of the slanted shape portion included on the body encircling opening side of the second welded part.

According to such an underpants-type absorbent article, the slanted shape portion of the fourth welded part provided to the end area is formed with a gentle inclination with respect to the slanted shape portion of the second welded part provided in the middle area. Therefore, the above described fourth welded part can gently block the tearing momentum that acts along the above described tearing direction.

It is preferable that in an underpants-type absorbent article, the slanted shape portion included on the body encircling opening side of the fourth welded part is provided along the slanted shape portion included on the body encircling opening side of the third welded part.

According to such an underpants-type absorbent article, the above described slanted shape portion included in the fourth welded part in the end area on the body encircling opening side is provided along the above described slanted shape portion included in the third welded part in the middle area on the body encircling opening side. Therefore, the guiding of the tearing direction to the shortest tearing route based on the third welded part in the middle area can be quickly taken over by the fourth welded part in the end area. Hereby, the above described guiding can swiftly continue in also this end area and as a result, this end part can be further certainly torn along the shortest tearing route.

It is preferable that in an underpants-type absorbent article, the slanted shape portion included on the body encircling opening side of the fourth welded part has a shape same as that of the slanted shape portion included on the body encircling opening side of the third welded part, and the fourth welded part and the third welded part are provided to be arranged along the horizontal direction while being inclined away from the horizontal direction such that the mutual slanted shape portions are aligned in a straight line.

According to such an underpants-type absorbent article, the guiding of the tearing direction to the shortest tearing route based on the third welded part in the middle area can be quickly taken over by the fourth welded part in the end area. Hereby, the above described guiding can swiftly continue in also this end area and as a result, this end part can be furthermore certainly torn along the shortest tearing route.

It is preferable that in an underpants-type absorbent article, when the welded part that is formed in the inner area and that includes the slanted shape portion is regarded as the first welded part,
a plurality of the first welded parts are formed in the inner area to be arranged in the vertical direction along an entire vertical length from the body encircling opening to the leg encircling opening.

According to such an underpants-type absorbent article, the force imparted by the person's hand is guided quickly and naturally along the aforementioned tearing direction along the entire vertical length in the inner area. In other words, the force is guided to change in the vertical direction toward the above described leg encircling opening as the force advances outside along the horizontal direction. Therefore, the above described end part can be quickly and easily torn.

It is preferable that in an underpants-type absorbent article,
the abdomen side part and the back side part respectively have an elastic member fixed along the horizontal direction in a state extended in the horizontal direction and
some of the welded parts are formed to a portion in the end part where the elastic member is not located.

According to such an underpants-type absorbent article, the strength of the above described several welded parts is reduced. Therefore, the above described end part can be torn easier by an amount the strength had been reduced. The reason for the strength of the above described several welded parts being reduced, that is, the reason for the reduced strength of the welded part formed to the portion where an elastic member is not positioned is as follows.

Firstly, the welded part is formed using a common pair of sandwiching members. To be specific, one sandwiching member includes a plurality of protrusions corresponding to the welding pattern and the other sandwiching member includes a flat surface that receives the protrusions. And a plurality of welded parts are formed by the pair of sandwiching members sandwiching the above described end part while the protrusions of the one sandwiching member being positioned to oppose the flat surface of the other sandwiching member. Here, the portion of the above described end part that does not have the elastic member positioned is thinner in the sandwiching direction compared to the portion where the elastic member is positioned by a thickness of the elastic member. Therefore, when the sandwiching takes place using the above described pair of sandwiching members, welding is difficult since the sandwiching pressure imparted becomes smaller by the amount of the thickness being reduced. As a result, the strength of the welded part formed to the portion where the elastic member is not positioned declines.

It is preferable that in an underpants-type absorbent article,
the abdomen side part and the back side part respectively have an elastic member fixed with hot-melt adhesive along the horizontal direction in a state extended along the horizontal direction and
some of the welded parts are formed by being provided with an ultrasound welding process to a portion in the end part where the elastic member is located.

According to such an underpants-type absorbent article, the strength of the above described several welded parts is reduced. For such reason, the above described end part can be easily torn by an amount of the strength reduced.

The reason for the strength of the above described several welded parts declining is as follows. Firstly, the portion where the above described several welded parts are formed in the above described end part has the elastic member fixed with hot-melt adhesive. However, when ultrasound welding is processed to the portion where this hot-melt adhesive exits, friction heating of the process by the ultrasound welding is suppressed by the lubricating action of this adhesive. Hereby, this welding process is made difficult which in turn reduces the strength of the above described welded part.

### ===Present Embodiment===

Fig. 2 is a schematic perspective view of the underpants-type disposable diaper 1 shown as an example of the underpants-type absorbent article according to the present embodiment. Fig. 3A is a schematic planar view of the opened state diaper 1 seen from the skin side. Fig. 3B is a schematic view taken along line B-B in Fig. 3A. Fig. 4 is a schematic exploded perspective view of the diaper 1 in an opened state. Fig. 5 is a schematic planar view of an opened state outer covering sheet 11 seen from the non-skin side.

In the following description, the side to be positioned on the skin side of the wearer when the diaper 1 is worn by the wearer is simply called the "skin side" whereas the side to be positioned on the non-skin side of the wearer is simply called the "non-skin side."

As illustrated in Fig. 3A, this diaper 1 is, for example, a two-pieced type diaper 1. In other words, this diaper 1 includes as the first component an absorbent main body 3 in for example, a substantially rectangular shape in plane view, which absorbs excreted liquid such as urine. And this diaper 1 includes as the second component an outer covering sheet 11 in a substantially hourglass shape in plane view, which is provided to cover the non-skin side face of the above described absorbent main body 3 to form the outer covering of the diaper 1.

As illustrated in Figs. 3A, 3B and 4, the absorbent main body 3 includes an absorbent core 3c that absorbs excreted liquid. The absorbent core 3c is made using liquid absorbent fiber such as pulp fiber or liquid absorbing particles such as high-absorbent polymer and the like which is formed in a substantially hourglass shape in plane view being an example of a predetermined shape. This absorbent core 3c may be covered with a liquid permeable covering sheet such as tissue paper and the like according to need.

A liquid permeable top sheet 4 such as nonwoven fabric is provided to the skin side face of this absorbent core 3c in a manner covering the entire face thereof. And similarly, a liquid impermeable leak-proof sheet 5 such as a film is provided to the non-skin side face of this absorbent core 3c in a manner covering the entire face thereof. In this example, both sheets 4, 5 are formed in a substantially rectangular shape in plane view and both sheets 4, 5 extend outward from the outer contour of the absorbent core 3c along the entire circumference thereof. And the portion of the top sheet 4 that is extending out and the portion of the leak proof sheet 5 that is extending out are joined together with adhesives, welding or the like. And as a result, an absorbent main body 3 in a substantially rectangular shape in plane view is formed.

Although not shown, an elastic string may be provided as the elastic member along the lengthwise direction of this absorbent main body 3 to the respective width direction end parts of this absorbent main body 3. This elastic string is for imparting elasticity to the portions proximate the leg encircling opening 1HL in the absorbent main body 3 and the outer covering sheet 11. Therefore, while this elastic string is inserted between the top sheet 4 and the leak proof sheet 5, the elastic string is extended from its natural length to a predetermined ratio of, for example, two to four times the natural length to be fixed in the extended state to the both sheets 4, 5 with adhesive such as hot-melt adhesives.

Further, a leak prevention wall part (not shown) for the purpose of preventing urine from leaking from the sides may be provided to the absorbent main body 3, depending on the case. This leak prevention wall part is also called a barrier cuff and is provided to stand on both end parts of the skin side face and the like of the absorbent main body 3 using a flexible sheet such as a nonwoven sheet as the material. Since this leak prevention wall part is a well known configuration, further explanation thereof will be omitted.

The outer covering sheet 11 is a soft sheet in a substantially hourglass shape in plane view when in an opened state as illustrated in Fig. 5. And this sheet 11 has three directions that are orthogonal to each other being the thickness direction, the lengthwise direction and the width direction. Further this outer covering sheet 11 is sectioned into three portions 11a, 11b, and 11c with respect to the lengthwise direction. In other words the outer covering sheet 11 is sectioned into the abdomen side part 11a which is to be positioned on the abdomen side of the wearer, the back side part 11b which is to be positioned on the back side of the wearer and the crotch part 11c which is to be positioned at the crotch of the wearer. It is a matter of course that the crotch part 11c is located between the abdomen side part 11a and the back side part 11b. And hereby, the crotch part 11c is a portion 11c that is formed constricted in the width direction of the substantially hourglass shape in plane view.

As illustrated in Fig. 4A, this outer covering sheet 11 has a single layer sheet 12 as the main body or a layered sheet having a plurality of sheets layered and integrated as the main body. And in this example, a single layer sheet 12 is used as the main body. Nonwoven fabric including thermoplastic resin fiber, thermoplastic resin film or the like is used as the material of this sheet 12 forming the main body. In this example, SMS nonwoven fabric (Spunbond - Meltblown - Spunbond nonwoven fabric) having a basis weight of 15 g/m² is used, however, there is no limitation to such.

Further, as illustrated in Figs. 4 and 5, this example has other sheets 13a, 13b joined to the skin side face of the sheet 12 by adhering, welding or the like the sheets to the abdomen side part 12a and the back side part 12b, respectively, of this sheet 12. But there is no other sheet joined to the crotch part 12c. However, there is no limitation to such and another sheet may be joined to also the crotch part 12c and further, this other sheet of the crotch part 12c may be made integral with the other sheet 13a of the abdomen side part 12a and the other sheet 13b of the back side part 12b. Furthermore, nonwoven fabric including thermoplastic resin fiber, thermoplastic resin film or the like is used as the material of these other sheets 13a, 13b. Spunbond nonwoven fabric including thermoplastic resin fiber and having a basis weight of 17 g/m² is used in this example, however, there is no limitation to such.

In the following description, the above described sheet 12 which forms the main body of the outer covering sheet 11 is called the "main body sheet 12", the above described other sheet 13a which is joined to the abdomen side part 12a of the main body sheet 12 is called the "abdomen side part outer surface sheet 13a" and the above described other sheet 13b which is joined to the back side part 12b of the main body sheet 12 is called the "back side part outer surface sheet 13b."

As illustrated in Figs. 4 and 5, elastic strings 15, 15 ... as an example of the elastic members are inserted between the main body sheet 12 and the abdomen side part outer surface sheet 13a and between the main body sheet 12 and the back side part outer surface sheet 13b. These elastic strings 15, 15 ... are for imparting in the width direction elasticity to the abdomen side part 11a and the back side part 11b of the outer covering sheet 11. Therefore, the elastic strings 15, 15 ... are extended from its natural length to a predetermined ratio of, for example, two to four times the natural length to be fixed in the extended state while being positioned along the width direction to the opened sheets 12, 13a, 13b with hot-melt adhesives. A plurality of these elastic strings 15, 15 ... is positioned in a manner arranged in the lengthwise direction of the main body sheet 12 with intervals therebetween. Hereby, elasticity is imparted along a wide area in the above described lengthwise direction to both the abdomen side part 11a and the back side part 11b.

As illustrated in Fig. 4, the abdomen side part outer surface sheet 13a and the back side part outer surface sheet 13b are respectively provided to extend out from the lengthwise end edge parts 12eLa, 12eLb of the main body sheet 12. And of the sheets 13a, 13b, the portions 13ap, 13bp that are extending out from the main body sheet 12 are folded back inward along the lengthwise direction. Hereby, the extended out portions 13ap, 13bp are laid over the skin side face of the main body sheet 12, as illustrated in Fig. 3B. And in this way, the end edge parts 11HBa, 11HBb that become the body encircling opening 1HB are formed by the fold-back part 13ak of the abdomen side part outer surface sheet 13a and the fold-back part 13bk of the back side part outer surface sheet 13b, in other words, the end edge parts 12eLa, 12eLb of the main body sheet 12 are covered with these fold-back parts 13ak, 13bk. Therefore, the wearer's skin is kept from being hurt with the end edge parts 12eLa, 12eLb of the main body sheet 12. As is clear by referring to Figs. 3A and 3B, this example has the above described extended out portions 13ap, 13bp that are laid over the skin side face of the main body sheet 12 further cover the lengthwise end parts 3ea, 3eb of the absorbent main body 3, however there is not limitation to such. In other words, the extended out portions 13ap, 13bp need not cover the end parts 3ea, 3eb.

As illustrated in Figs. 3A and 4, the aforementioned absorbent main body 3 is fixed at the center position in the width direction of this skin side face of the outer covering sheet 11, that is, the skin side face of the main body sheet 12 while their lengthwise directions are aligned with each other. Further, the lengths of the absorbent main body 3 in the lengthwise direction and the width direction are respectively of a length such that this absorbent main body 3 is substantially contained inside the outer covering sheet 11.

And as illustrated in Fig. 3A, the outer covering sheet 11 to which the absorbent main body 3 is fixed is folded into two at the crotch part 11c so that the abdomen side part 11a overlaps the back side part 11b. In this overlapping state, the abdomen side part 11a and the back side part 11b are respectively welded at the width direction end parts 11es. Hereby, an underpants-type diaper 1 is formed with a body encircling opening 1HB and a pair of leg encircling openings 1HL, 1HL as illustrated in Fig. 2.

The width direction end part 11es that is welded in the aforementioned manner is also called the "side end part 11es" in the following description.

The following defines the directions of the three dimensionally shaped diaper 1 made in an underpants-type as illustrated in Fig. 2. Firstly, the direction parallel to the width direction of the outer covering sheet 11 is called the "horizontal direction." And the direction parallel to the lengthwise direction of the outer covering sheet 11 is called the "vertical direction." The horizontal direction is orthogonal to the vertical direction as an example of intersection. And the direction that is orthogonal to both the horizontal direction and the vertical direction is called the "front-rear direction." The front in the front-rear direction is the abdomen side being the front side of the wearer and the rear of the front-rear direction is the back side being the rear side of the wearer. Further, since the vertical direction is directed along the up-down direction when the diaper 1 is worn, the vertical direction is also called the "up-down direction." When the up-down direction is used in the description, the leg encircling opening 1HL is positioned below the body encircling opening 1HB. Further, the center side in the horizontal direction is also called the "inner side" and the end side in the horizontal direction is also called the "outer side." For example, the above described side end part 11es is provided at a location on the outer side along the horizontal direction of the diaper 1 and the above described absorbent main body 3 is provided to a location on the inner side along the horizontal direction of the diaper 1.

The aforementioned welding is performed by such as an ultrasound welding process or a heat seal process. And this example employs the ultrasound welding process. With this welding, the horizontal edge parts 11es at the abdomen side part 11a of the outer covering sheet 11 in Fig. 2, that is, each of the side end part 11es have formed thereto a plurality of welded parts j, j ... based on a predetermined welding pattern. And similarly, each of the side end part 11es in the back side part 11b of the outer covering sheet 11 also have formed thereto a plurality of welded parts j, j ... (not shown in Fig. 2) based on the same welding pattern. Here, the welded parts j, j ... of the abdomen side part 11a and the welded parts j, j ... of the back side part 11b are made integral and are positioned to be in a front-back relation in the above mentioned side end parts 11es, so to speak, being the same items. Therefore, the two are not differentiated in the following description.

Figs. 6 to 7B are explanatory views of the welding pattern. Fig. 6 indicates the side end part 11es enlarged and Figs. 7A and 7B respectively indicate the upper part of a welding pattern further enlarged.

The welding pattern is in a strip form long in the vertical direction as a whole. And in the example shown in Fig. 6A, the welding pattern extends along substantially the entire length of the side end part 11es from the body encircling opening 1HB to the leg encircling opening 1HL. This pattern is in a non-continuous welding pattern with respect to each of the vertical and horizontal directions. Hereby, the plurality of welded parts j, j ... are disposed with spaces between the welded parts j, j ... that are adjacent in both the vertical direction and the horizontal direction. Further, each of the welded parts j are formed depressed along the front-back direction (the direction penetrating the plane of the paper in Fig. 6), and further in this example, the bottom surfaces of the welded parts j are formed in a parallelogram long in the horizontal direction.

As illustrated in Fig. 6 and 7A, the side end part 11es includes three areas Ain, Amid, Aout in vertically long strip forms that are arranged in the horizontal direction. To be specific, the side end part 11es includes an inner area Ain in a vertically long strip form positioned on the inner side along the horizontal direction, a middle area Amid in a vertically long strip form positioned on the outer side along the horizontal direction with respect to the inner area Ain, and an end area Aout in a vertically long strip form positioned on the outer side along the horizontal direction with respect to the middle area Amid. And the horizontal dimensions of these three areas Ain, Amid, Aout are substantially the same. Further, the area Amid, Aout configured by combining the middle area Amid and the end area Aout corresponds to the "outer area" in the claims, that is, corresponds to the "outer area outer area being located horizontally outward with respect to the inner area."

Since the welded parts j, j ... formed to the areas Ain, Amid, Aout are differentiated in the following description, the welded part j provided to the inner area Ain is called the "first welded part j1", the two types of welded parts j, j as an example of the plural types of welded parts j, j provided to the middle area Amid are respectively called the "second welded part j2" and the "third welded part j3", and the welded part j provided to the end area Aout is called the "fourth welded part j4."

A plurality of first welded parts j1 is provided arranged vertically in a row in the inner area Ain. And each welded part j1 in a parallelogram is provided to slant downward in the vertical direction as approaching to the outside along the horizontal direction. Hereby, the top side j1u (corresponding to the slanted shape portion) of the first welded part j1 is in a slanted shape such that the slanting portion slants downward in the vertical direction as approaching to the outside along the horizontal direction. This slanted shape comes along the aforementioned tearing direction (see Fig. 1A.) In other words, the term "tearing direction" used here indicates the direction in which the side end part 11es is torn when the side end part 11es is torn from the inner side toward the outer side along the horizontal direction by a person applying a force F to the body encircling opening 1HB that is positioned to the upper end part 11esu of the side end part 11es as has been mentioned above with reference to Fig. 1A. However, this tearing direction is directed downward in the vertical direction as approaching to the outside along the horizontal direction. And corresponding to such, the above described slanted shape is also directed toward the same direction. Therefore, the first welded parts j1 can quickly guide the force F, applied to the body encircling opening 1HB and facing outward along the horizontal direction so to be directed toward the direction along the above described tearing direction based on this slanted shape. Hereby, this side end part 11es can be torn easily from the inner side toward the outer side along the horizontal direction.

The first welded parts j1 of the inner area Ain are arranged in the vertical direction such that a wide space Ga and a narrow space Gb are present alternately in the vertical direction. In this example, the vertical dimensions of the wide spaces Ga are set such that all the wide spaces Ga, Ga ... have the same dimensions. Similarly, the vertical dimension of the narrow spaces Gb is set such that all the narrow spaces Gb, Gb ... have the same dimensions. Further in this example, the vertical arrangement pitch Pa of the pair of welded parts j1, j1 provided to sandwich the wide space Ga is set to be twice the vertical arrangement pitch Pb of the pair of welded parts j1, j1 provided to sandwich the narrow space Gb. However, there is no limitation to such. For example, the pitch size may be made, for example, three times or other integral multiples or the pitch size may be made, for example, 1.5 times being a multiple including a decimal point.

In the present embodiment, the upper edge part of the inner area Ain has the wide space Ga corresponding thereto. In other words, this upper edge part is provided with a single first welded part j1 together with a first welded part j1 provided below the above welded part j1 with a wide space Gb therebetween. And this enables the start of the tearing to be performed smoothly. In other words, as mentioned above, the start of the tearing is important in order to smoothly tear the entire side end part 11es. And for a smooth tearing movement at the start of tearing the force F required at the start of the tearing needs to be small, and with respect to this, a wide space Ga is made to correspond to the upper end part of the inner area Ain in this example. Hereby, the force F required at the start of tearing can be kept small thereby allowing a smooth tearing movement at the start of tearing.

The inclination angle θ1, at the top side j1u and the bottom side j1d of this first welded part j1, formed between the horizontal direction is selected from the range of, for example, 10 to 40 degrees and preferably selected from a range of 15 to 35 degrees, and more preferably selected from a range of 20 to 25 degrees. And in this example, the inclination angle is selected to be 22 degrees.

The horizontal dimension of the bottom surface of the first welded part j1 is selected such that an arbitrary value takes a maximum value from, for example, 1 mm to 4 mm. Similarly, the vertical dimension thereof is selected such that an arbitrary value takes a maximum value from, for example, 0.5 mm to 1.5 mm. The vertical dimension of the above described wide space Ga is selected from, for example, 5 mm to 10 mm, and the vertical dimension of the above described narrow space Gb is selected from, for example, 1 mm to 4 mm. However, there is no limitation to such.

Meanwhile, the middle area Amid outwardly adjacent to the inner area Ain, as mentioned above, has as an example of a plurality of types the second welded part j2 and the third welded part j3 provided to be arranged vertically in a row.

The second welded part j2 is a welded part that has a shape substantially same as that of the aforementioned first welded part j1. In other words, the specification of the second welded part j2 is the same as that of the first welded part j1 other than the horizontal dimension thereof being about ten to twenty percent smaller than the first welded part j1. To be more specific, this second welded part j2 also has a parallelogram bottom surface. And the top side j2u and the top side j2u (both correspond to the slanted shape portion) of this second welded part j2 are formed in a slanted shape such that the slanting portion slants downward in the vertical direction as approaching to the outside along the horizontal direction. Further, the inclination angle θ2, at the top side j2u and the bottom side j2d, formed between the horizontal direction takes the same value as the inclination angle θ1 of the aforementioned first welded part j1.

And the second welded part j2 is basically provided to respectively correspond to the first welded parts j1 in the inner area Ain. In other words, the second welded part j2 is positioned to be arranged horizontally outward the first welded part j1 while the top side j2u of the second welded part j2 being inclined away from the horizontal direction so to align in a straight line with the top side j1u of the first welded part j1.

Hereby, the guiding of the aforementioned force toward the tearing direction by the first welded part j1 in the inner area Ain can be quickly taken over by the second welded part j2 in the middle area Amid and thus the above described guiding can be quickly taken over in also this middle area Amid. And as a result, this side end part 11es can be easily torn.

Additionally, when the second welded parts j2 are provided to the respective first welded parts j1 in the inner area Ain as mentioned above, these second welded parts j2 are arranged in the vertical direction such that a wide space Ga and a narrow space Gb are present alternately in the vertical direction, being basically the same as in the case of the first welded part j1. However in this example, another second welded part j2 is additionally provided at a location vertically in the middle of the wide space Ga and hereby this wide space Ga is vertically divided into two, that is, two narrow spaces Gb, Gb are formed vertically adjacent each other. Therefore, the plurality of second welded parts j2, j2 ... are provided with a small vertical arrangement pitch Pb corresponding to the aforementioned narrow space Gb, in the middle area Amid. Further, these two narrow spaces Gb, Gb each have provided thereto a third welded part j3, respectively.

Therefore, the portion corresponding to the wide space Ga in the middle area Amid that is horizontally adjacent and corresponding to the wide space Ga in the inner area Ain has provided one second welded part j2 and two third welded parts j3, j3. Hereby, the wide space Ga in the vertical direction is divided. And as a result, the dimension of the space between the vertically adjacent welded parts j , j is larger at the inner area Ain than that at the middle area Amid.

And this holds true for the upper end part 11esu of the side end part 11es. In other words, as mentioned above, the upper edge part of the inner area Ain has a wide space Ga positioned to correspond thereto and the portion corresponding to the wide space Ga of the middle area Amid positioned on the side thereof has one second welded part j2 and two third welded parts j3, j3 provided. Hereby, the wide space Ga is sectioned into four extremely narrow spaces. And as a result, the vertical dimension of the space between the welded parts j1, j1 in the inner area Ain is made larger than the vertical dimension of the space between the welded parts j2, j3 in the middle area Amid. Since the above described dimension of the space in the inner area Ain is large, tearing at the start from the body encircling opening 1HB at the upper end part 11esu can be carried out with an excellent tearing performance while the strength of the upper end part 11esu is effectively reinforced based on the above described space in the middle area Amid being small. Hereby, the side end part 11es can be kept from being inevitably torn at the upper end part 11esu when wearing the diaper.

By the way, as illustrated in Fig. 7B, the third welded part j3 is also a welded part that has a parallelogram bottom surface. However, the slanted shape of the top side j3u (corresponding to the slanted shape portion) of this third welded part j3 has a shape slanting reverse the first welded part j1. In other words, the top side j3u of this third welded part j3 is formed in a slanted shape such that the slanting portion slants upward in the vertical direction as approaching to the outside along the horizontal direction. Further, the inclination angle θ3, at the top side j3u, formed between the horizontal direction takes a different value from the aforementioned inclination angle θ1 of the aforementioned first welded part j1, and in this example, the inclination angle θ3 of the third welded part j3 takes a smaller value.

Meanwhile, as illustrated in Fig. 7B, the second welded part j2 is located vertically above and adjacent the third welded part j3. And since this second welded part j2 has a parallelogram bottom surface in this example, the bottom side j2d thereof is parallel to the top side j2u thereof. Therefore, this bottom side j2d is also formed in a slanted shape such that the slanting portion slants downward in the vertical direction as approaching to the outside along the horizontal direction.

In this way, the middle area Amid has the second welded part and the third welded part provided to associate with each other to partition a taper shaped area A23 that has the portions horizontally outward thereof vertically narrowed. And since this area A23 is a so-called non-welded part that is not welded, the strength thereof is lower than the welded parts j2, j3. Hereby, the tearing direction is guided to advance through this taper shaped area A23. And as a result, this tearing direction is changed to take a route that is substantially parallel to the horizontal direction, as illustrated in Fig. 7B. And hereby, the above described side end part 11es can be torn taking the shortest route which comes closest to the horizontally outward edge part 11es1 at this side end part 11es and thus this side end part 11es can be torn easily.

The inclination angles θ3, at the top side j3u and the bottom side j3d of this third welded part j3, formed between the horizontal direction are selected from the range of, for example, 5 to 30 degrees and preferably selected from a range of 5 to 15 degrees, and more preferably selected from a range of 7 to 12 degrees. And in this example, the inclination angle is selected to be 10 degrees. Further, the horizontal dimension of the bottom surface of the third welded part j3 is selected such that an arbitrary value takes a maximum value from, for example, 1 mm to 4 mm. Similarly, the vertical dimension thereof is selected such that an arbitrary value takes a maximum value from, for example, 0.5 mm to 1.5 mm.

As illustrated in Fig. 7B, the end area Aout outwardly adjacent to the middle area Amid has a plurality of fourth welded parts j4, j4 ... provided to be arranged vertically. And in this example, the fourth welded part j4 is a welded part that has a shape substantially the same as that of the aforementioned third welded part j3. In other words, the specification of the fourth welded part j4 is the same as that of the third welded part j3 other than the horizontal dimension thereof being about ten to twenty percent larger than the third welded part j3. To be more specific, this fourth welded part j4 also has a parallelogram bottom surface. And the top side j4u and the bottom side j4d of this fourth welded part j4 are formed in slanted shapes such that the slanting portion slants upward in the vertical direction as approaching to the outside along the horizontal direction. Further, this inclination angles θ4, at the top side j4u and the bottom side j4d, formed between the horizontal direction takes the same value as the inclination angle θ3 of the aforementioned third welded part j3.

And the fourth welded parts j4 are basically provided to respectively correspond to at least the third welded parts j3 in the middle area Amid. In other words, the fourth welded parts j4 are positioned to be arranged horizontally outward the third welded part j3 while the top side j4u (corresponding to the slanted shape portion) of the fourth welded part j4 being inclined away from the horizontal direction so to align in a straight line with the top side j3u of the third welded part j3.

Hereby, the guiding of the force to take the shortest route toward the tearing direction based on the third welded part j3 in the middle area Amid can be quickly taken over by the fourth welded part j4 in the end area Aout. Thus the above described guiding can be quickly taken over in also this end area Aout. And as a result, this side end part 11es can be further certainly torn along the shortest route.

As illustrated in Fig. 7B, this fourth welded part j4 intersects with the line extending from the top side j2u of the second welded part j2 in the middle area Amid. Therefore, the momentum of the tearing along the tearing direction that is created at the inner area Ain and the middle area Amid when tearing the side end part 11es is blocked and thus the tearing momentum can be relieved. As a result, a person can horizontally tear the side end part 11es with an appropriate momentum. By the way, in the example of Fig. 7B, the fourth welded parts j4 are provided to correspond to all the second welded parts j2, j2 ... included in the middle area Amid. Therefore, the above described action to relieve the tearing momentum can be certainly performed.

Further, the inclination angle θ4, at the top side j4u of the fourth welded part j4, formed with the horizontal direction takes a value that is smaller than the inclination angle θ2, of the top side j2u of the second welded part j2, formed between the horizontal direction. Therefore, the fourth welded part j4 can gently block the momentum along the above described tearing direction.

In the aforementioned example, the welding pattern included four types of welded parts j being the first to the fourth welded parts j1, j2, j3, j4, however there is no limitation to such. In other words, a welded part j of a different type may be further added or one or two types of the above described second to fourth welded parts j2, j3, j4 may be omitted. For example, the following may be employed. The example illustrated in Fig. 8A shows a case where the first welded parts j1, the second welded parts j2 and the third welded parts j3 are included but the fourth welded parts j4 are not included. Further the example illustrated in Fig. 8B shows a case where the first welded parts j1 and the second welded parts j2 are included but the third welded parts j3 and the fourth welded parts j4 are not included. Furthermore, the example illustrated in Fig. 8C shows a case where the first welded parts j1, the second welded parts j2 and the fourth welded parts j4 are included but the third welded parts j3 are not included.

And as in the example illustrated in Fig. 9A, a first welded part j1 and one second welded part j2 closest thereto may be connected to form a single welded part j12, or as in the example illustrated in Fig. 9B, a third welded part j3 and one fourth welded part j4 closest thereto may connected to form a single welded part j34. However, when such connection takes place, the strength of the welded parts j12, j34 becomes strong which in turn deteriorates the tearing performance. For such reason, it is preferable that a connection is not made.

By the way, in this example, the abdomen side part 11a and the back side part 11b respectively have elastic strings 15, 15 ... fixed along the horizontal direction with hot-melt adhesive in a state extended along the horizontal direction, as illustrated in Figs. 4 and 5. And as illustrated in Fig. 10, some of the welded parts ja, ja ... among the welded parts j, j ... are formed by being provided with an ultrasound welding process to a portion where the elastic string 15 is located in the side end part 11es. Hereby, the strength of the above described some of the welded parts ja, ja ... become weak. Thus the tearing at the side end part 11es becomes easier by an amount the strength declines. For such reason, this configuration is preferable from the viewpoint of the tearing performance. The reason for the above described strength of some of the welded parts ja declining is as follows.

Firstly, the portion where the above described some of the welded parts ja are formed in the side end part 11es has the elastic string 15 fixed with hot-melt adhesive. However, when an ultrasound welding process is performed to the portion where this hot-melt adhesive exists, friction heating of the process by the ultrasound welding is suppressed by the lubricating action of this adhesive. Hereby, this welding process is made difficult which in turn reduces the strength of the above described welded part ja, as a result.

Meanwhile, even in the case where the elastic strings 15, 15 ... are fixed to the abdomen side part 11a and the back side part 11b by welding, the strength of the welded part jb formed to the portion where the elastic string 15 does not exist in the side end part 11es becomes smaller than the strength of the welded part ja formed to the portion where the elastic string 15 exists, as illustrated in Fig. 10. For such reason, it is preferable in this case that the welded part jb is formed to a portion in the side end part 11es where the elastic string 15 does not exist from the viewpoint of tearing performance. By the way, the reason for the reduced strength of the welded part jb formed to the portion where the elastic string 15 does not exist is as follows.

Firstly, the welded parts j, j ... are formed using a pair of sandwiching members which is not shown. To be specific, one sandwiching member includes a plurality of protrusions corresponding to the welding pattern and the other sandwiching member includes a flat surface that receives the protrusions. And a plurality of welded parts j, j ... are formed by the entire vertical length of the side end part 11es being sandwiched from both the front and the rear directions by these sandwiching members while the protrusions of the one sandwiching member being positioned to oppose the flat surface of the other sandwiching member. Here, the portion of the above described side end part 11es that does not have the elastic member 15 positioned is thinner in the above described front-rear direction being the sandwiching direction compared to the portion where the elastic member 15 is positioned by an amount of the elastic member 15. Therefore, when the sandwiching takes place using the above described pair of sandwiching members, welding is difficult since the sandwiching pressure imparted becomes smaller by the amount of the thickness being reduced. As a result, the strength of the welded part jb formed to the portion where the elastic member 15 is not positioned declines.

Fig. 11 is an explanatory view of a modified example of the welding pattern. As illustrated in Fig. 6, the aforementioned embodiment had the welded parts j , j , ... formed in the same pattern along the entire vertical length of the side end part 11es, however, there is no limitation to such. For example, this modified example has the side end part 11es sectioned into two areas Au, Ad one above the other with respect to the vertical direction. And the area above Au and the area below Ad have welded parts j, j ... formed in welding patterns that are different from each other. In the following, the area above Au is called the "upper area Au" and the area below Ad is called the "lower area Ad."

As illustrated in Fig. 11, the upper area Au has a length that starts from the upper end part 11esu of the side end part 11es and extends downward and this length is substantially two thirds of the entire length of the side end part 11es. On the other hand, the lower area Ad has a length that starts from the lower end part 11esd of the side end part 11es and extends upward and this length is substantially one third of the entire length of the side end part 11es. And the welding pattern in the upper area Au is the same as the aforementioned welding pattern (Fig. 6), however, the welding pattern in the lower area Ad is different from the aforementioned welding pattern (Fig. 6.) Therefore, the following describes only the welding pattern in the lower area Ad and explanation of the welding pattern in the upper area Au is omitted.

As illustrated in Fig. 11, the lower area Ad also includes an inner area Ain, a middle area Amid and an end area Aout that are arranged in the horizontal direction. And in the inner area Ain, a plurality of the fifth welded parts j5, j5 ... that are similar to the first welded part j1 are vertically formed in a predetermined arrangement pitch. And the middle area Amid has formed a plurality of the sixth welded parts j6, j6 ... that are similar to the second welded part j2 and a plurality of the seventh welded parts j7, j7 ... that are similar to the third welded part j3 formed alternatively along the vertical direction in a predetermined arrangement pitch. Further, the end area Aout has formed a plurality of the eighth welded parts j8, j8 ... that are similar to the fourth welded part j4 vertically formed in a predetermined arrangement pitch. Furthermore, the fifth to the eighth welded parts j5, j6, j7, j8 all have a parallelogram bottom surface similar to the aforementioned first to the fourth welded parts j1, j2, j3, j4.

Here, the fifth welded part j5 in the inner area Ain slants downward in the vertical direction as approaching to the outside along the horizontal direction. Hereby, this fifth welded part j5 exhibits a similar operational advantage as the first welded part j1 in the upper area Au. However, in this modified example, the tearing performance is slightly sacrificed and the vertical dimension of the spaces between the fifth welded parts j5, j5 ... that are adjacent in the vertical direction in the lower area Ad are made smaller than the vertical dimension of the space between the first welded parts j1, j1 that are adjacent in the vertical direction in the upper area Au from the viewpoint of giving priority to keep the side end part 11es from tearing that starts from the leg encircling opening 1HL when the diaper is worn.

Further, the sixth welded part j6 in the middle area Amid is slanted downward in the vertical direction as approaching to the outside along the horizontal direction. Hereby, the sixth welded part j6 exhibits a similar operational advantage as the second welded part j2 in the upper area Au. Further, the seventh welded part j7 in the middle area Amid is slanted upward in the vertical direction as approaching to the outside along the horizontal direction. Hereby the seventh welded part j7 exhibits a similar operational advantage as the third welded part j3 in the upper area Au. However, based on the aforementioned viewpoint, the vertical dimension of the space between the sixth welded part j6 and the seventh welded part j7 that are adjacent in the vertical direction in the lower area Ad is made smaller than the vertical dimension of the space between the second welded part j2 and the third welded part j3 that are adjacent in the vertical direction in the upper area Au.

Further, the eight welded part j8 in the end area Aout slants upward in the vertical direction as approaching to the outside along the horizontal direction. Hereby, this eighth welded part j8 exhibits a similar operational advantage as the fourth welded part j4 in the upper area Au. However, the vertical dimension of the space between the eighth welded parts j8, j8 ... that are adjacent in the vertical direction in the lower area Ad are made smaller than the vertical dimension of the space between the fourth welded parts j4, j4 that are adjacent in the vertical direction in the upper area Au based on the aforementioned viewpoint.

### ===Other Embodiments===

The embodiments of the present invention has been described, however, the foregoing embodiments are intended to facilitate the understanding of the present invention but not to limit the invention. And it is needless to say that modifications and improvements of the present invention are possible without departing from the scope of the invention, and equivalents thereof are also encompassed by the invention. For example, the following modifications are possible.

The aforementioned embodiment indicated a horizontally long parallelogram as an example of the shape of the bottom surfaces of the first to the eighth welded parts j1, j2, ..., j8, however, there is no limitation to such. For example, a horizontally long rectangle may do or a horizontally long ellipse may do, and other shapes may do.

The aforementioned embodiment exemplified welded parts j (j1, j2, ... j8) including a parallelogram bottom surface and in connection to this, a top side and a bottom side in this parallelogram were exemplified as an example of the slanted shape portion included on the body encircling opening 1HB side and the leg encircling opening 1HL side. In other words, a linearly inclined slanted shape portion was exemplified, however, there is no limitation to such. For example, the welded part may include a slanted shape portion inclined in a curved line instead of the aforementioned linearly inclined slanted shape portion.

As illustrated in Fig. 3A, a two-pieced type diaper 1 configured with the absorbent main body 3 as the first component covered with an outer covering sheet 11 in a substantially hourglass shape in plane view as the second component had been exemplified in the aforementioned embodiment, however there is no limitation to such. For example, the aforementioned welded parts j (j1, j2, ... j8) according to the present invention may be formed to a so-called three-pieced type diaper 1a. Description thereof is as follows. Firstly, as illustrated in Fig. 12, this diaper 1a includes as the first component an absorbent main body 3 that is to be arranged to the crotch of a wearer, as the second component an abdomen side strip member 21a that is to be arranged to the abdomen side of this wearer, and as the third component a back side strip member 21b that is to be arranged to the back side of this wearer. Further, in the opened state illustrated in Fig. 12, the abdomen side strip member 21a and the back side strip member 21b are in a state arranged parallel with a space therebetween with the absorbent main body 3 being extended across between the two strip members and with the lengthwise end parts 3ea, 3eb of the absorbent main body 3 being respectively joined and fixed to the closest strip members 21a, 21b, and thereby an outside view is in a substantially H-shaped form in a plane view is made. And from this opened state, the absorbent main body 3 is folded in half at substantially the center in the lengthwise direction while the strip members 21a, 21b overlapping each other in this state folded in half. And the two strip members 21a, 21b are welded at the end parts 21es in the width direction (corresponding to the horizontal direction) that is orthogonal to the aforementioned lengthwise direction so that the strip members 21a, 21b are connected in an annular form. Hereby, a diaper 1a formed with a body encircling opening and a pair of leg encircling openings is configured. And the aforementioned welded parts j (j1, j2, ... j8) may be applied also to this welding. Here, the strip members 21a, 21b are formed using material of flexible sheets such as nonwoven fabric including thermoplastic resin fiber. Further, the configuration of the absorbent main body 3 is substantially the same as that aforementioned and thus description thereof is omitted.

### [Reference Signs List]

1 diaper (absorbent article), 1a diaper (absorbent article),
1HB body encircling opening, 1HL leg encircling opening,
3 absorbent main body, 3ea end part, 3eb end part,
3c absorbent core, 4 top sheet, 5 leak proof sheet,
11 outer covering sheet,
11HBa end edge part that becomes the body encircling opening,
11HBb end edge part that becomes the body encircling opening,
11a abdomen side part, 11b back side part, 11c crotch part,
11es side end part (end part), 11es1 edge part,
11esu upper end part, 11esd lower end part,
12 main body sheet, 12a abdomen side part, 12b back side part,
12c crotch part,
12eLa end edge part, 12eLb end edge part,
13a abdomen side part outer surface sheet,
13ak fold-back part, 13ap extended out portion,
13b back side part outer surface sheet,
13bk fold-back part, 13bp extended out portion,
15 elastic string (elastic member),
21a abdomen side strip member, 21b back side strip member,
21es end part,
Au upper area, Ad lower area,
Ga wide space, Gb narrow space,
j welded part,
j1 first welded part, j1u top side (slanted shape portion),
j1d bottom side,
j2 second welded part, j2u top side (slanted shape portion),
j2d bottom side (slanted shape portion),
j3 third welded part, j3u top side (slanted shape portion),
j3d bottom side,
j4 fourth welded part, j4u top side (slanted shape portion),
j4d bottom side,
j5 fifth welded part, j6 sixth welded part,
j7 seventh welded part, j8 eighth welded part,
j12 welded part, j34 welded part,
ja welded part, jb welded part,
Ain inner area, Amid middle area (outer area),
Aout end area (outer area)
A23 taper shaped area

## Claims

1. An underpants-type absorbent article configured with an absorbent article having an abdomen side part that is to be arranged on an abdomen side of a wearer, a back side part that is to be arranged on a back side of the wearer and a crotch part that is to be arranged to a crotch of the wearer when worn, the absorbent article being welded at respective horizontally outward end parts of the abdomen side part and the back side part when the absorbent article is in a state folded into two at the crotch part with the abdomen side part overlapping the back side part to form a body encircling opening and a pair of leg encircling openings at mutually different locations along a vertical direction that intersects the horizontal direction, the underpants-type absorbent article comprising:
a plurality of welded parts that are formed at the end part in a non-continuous welding pattern along at least the vertical direction; and
an inner area and an outer area that are included in the end part, the inner area being located horizontally inward and the outer area being located horizontally outward with respect to the inner area; wherein
the welded parts are formed to a predetermined range along the vertical direction, the predetermined range starting from the body encircling opening of the end part, such that a vertical dimension of a space between vertically adjacent welded parts in the inner area is made larger than the vertical dimension of a space between the vertically adjacent welded parts in the outer area, and
the welded parts each has on the body encircling opening side a slanted shape portion, the slanting portion slanting toward the leg encircling opening in the vertical direction as approaching to an outside along the horizontal direction, the welded parts being formed in pluralities and arranged along the vertical direction at the inner area in the predetermined range.

2. An underpants-type absorbent article according to claim 1, wherein
the outer area includes a middle area adjacent to the inner area, and when the welded part including the slanted shape portion is regarded as a first welded part,
a second welded part and a third welded part are provided to the middle area,
the second welded part includes on the leg encircling opening side the slanted shape portion, the slanting portion slanting toward the leg encircling opening in the vertical direction as approaching to an outside along the horizontal direction,
the third welded part includes on the body encircling opening side the slanted shape portion, the slanting portion slanting toward the body encircling opening in the vertical direction as approaching to an outside along the horizontal direction, and
the second welded part and the third welded part associate with each other to partition in the middle area, a taper shaped area that is narrowed in the vertical direction as approaching to the outside along the horizontal direction.

3. An underpants-type absorbent article according to claim 2, wherein
the second welded part includes on the body encircling opening side the slanted shape portion, the slanting portion slanting toward the leg encircling opening in the vertical direction as approaching to the outside along the horizontal direction and
the slanted shape portion is provided along the slanted shape portion included on the body encircling opening side of the first welded part.

4. An underpants-type absorbent article according to claim 3, wherein
the slanted shape portion included on the body encircling opening side of the second welded part has a shape same as that of the slanted shape portion included on the body encircling opening side of the first welded part, and the second welded part and the first welded part are provided to be arranged along the horizontal direction while being inclined away from the horizontal direction such that the mutual slanted shape portions are aligned in a straight line.

5. An underpants-type absorbent article according to any one of claims 2 to 4, wherein
the outer area includes an end area located horizontally outward with respect to the middle area,
the end area is provided with a fourth welded part,
the fourth welded part includes on the body encircling opening side the slanted shape portion, the slanting portion slanting toward the body encircling opening in the vertical direction as approaching to an outside along the horizontal direction, and
the fourth welded part is provided to intersect with a line extending from the slanted shape portion included on the body encircling opening side of the second welded part.

6. An underpants-type absorbent article according to claim 5, wherein
an inclination of the slanted shape portion of the fourth welded part is more gentle with respect to an inclination of the slanted shape portion included on the body encircling opening side of the second welded part.

7. An underpants-type absorbent article according to claim 5 or 6, wherein
the slanted shape portion included on the body encircling opening side of the fourth welded part is provided along the slanted shape portion included on the body encircling opening side of the third welded part.

8. An underpants-type absorbent article according to claim 7, wherein
the slanted shape portion included on the body encircling opening side of the fourth welded part has a shape same as that of the slanted shape portion included on the body encircling opening side of the third welded part, and the fourth welded part and the third welded part are provided to be arranged along the horizontal direction while being inclined away from the horizontal direction such that the mutual slanted shape portions are aligned in a straight line.

9. An underpants-type absorbent article according to any one of claims 1 to 8, wherein
when the welded part that is formed in the inner area and that includes the slanted shape portion is regarded as the first welded part,
a plurality of the first welded parts are formed in the inner area to be arranged in the vertical direction along an entire vertical length from the body encircling opening to the leg encircling opening.

10. An underpants-type absorbent article according to any one of claims 1 to 9, wherein
the abdomen side part and the back side part respectively have an elastic member fixed along the horizontal direction in a state extended in the horizontal direction and
some of the welded parts are formed to a portion in the end part where the elastic member is not located.

11. An underpants-type absorbent article according to any one of claims 1 to 9, wherein
the abdomen side part and the back side part respectively have an elastic member fixed with hot-melt adhesive along the horizontal direction in a state extended along the horizontal direction and
some of the welded parts are formed by being provided with an ultrasound welding process to a portion in the end part where the elastic member is located.
